(19) Europäisches Patentamt

European Patent Office

Office européen des brevets

(11) **EP 2 632 470 B1**

(12) **EUROPEAN PATENT SPECIFICATION**

(45) Date of publication and mention of the grant of the patent:
**28.02.2018 Bulletin 2018/09**

(21) Application number: **11781931.8**

(22) Date of filing: **28.10.2011**

(51) Int Cl.:
*A61K 31/555* (2006.01)    *A61Q 5/00* (2006.01)
*A61Q 7/00* (2006.01)    *A61Q 17/00* (2006.01)
*A61K 8/34* (2006.01)    *A61K 8/365* (2006.01)
*A61K 8/41* (2006.01)    *A61K 8/46* (2006.01)
*A61K 8/49* (2006.01)

(86) International application number:
**PCT/US2011/058313**

(87) International publication number:
**WO 2012/058557 (03.05.2012 Gazette 2012/18)**

(54) **PERSONAL CARE COMPOSITIONS COMPRISING A PYRITHIONE AND AN IRON CHELATOR**

KÖRPERPFLEGEZUSAMMENSETZUNGEN MIT EINEM PYRITHION UND EINEM EISENCHELATOR

COMPOSITIONS DE SOINS PERSONNELS COMPRENANT UNE PYRITHIONE ET UN CHÉLATEUR DU FER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2010 US 407754 P**

(43) Date of publication of application:
**04.09.2013 Bulletin 2013/36**

(73) Proprietor: **The Procter & Gamble Company**
**Cincinnati, OH 45202 (US)**

(72) Inventors:
• **SCHWARTZ, James, Robert**
**West Chester**
**Ohio 45069 (US)**
• **SAUNDERS, Charles, Winston**
**Fairfield**
**Ohio 45014 (US)**
• **YOUNGQUIST, Robert, Scott**
**Mason**
**Ohio 45040 (US)**
• **KELLY, Casey, Patrick**
**Wyoming**
**Ohio 45215 (US)**
• **DOMSIC, Jeffrey, Kenneth**
**Liberty Twp.**
**Ohio 45044 (US)**
• **LUCAS, Robie, Lynn**
**Liberty Twp.**
**Ohio 45011 (US)**
• **XU, Jun**
**Mason**
**Ohio 45040 (US)**

(74) Representative: **Sauvaître, Thibault Bruno**
**Procter & Gamble Service GmbH**
**Patent Department**
**PO Box 14**
**Frankfurter Straße 145**
**61476 Kronberg im Taunus (DE)**

(56) References cited:
EP-A2- 0 158 481    EP-A2- 0 268 911
WO-A1-2009/053163    WO-A2-2007/106622
WO-A2-2011/056667    FR-A1- 2 758 720
US-A- 4 470 982    US-A- 5 968 539

**Description**

FIELD OF THE INVENTION

The present invention is directed to personal care compositions comprising a pyrithione or a polyvalent metal salt of a pyrithione and an effective amount of an iron chelator.

BACKGROUND OF THE INVENTION

Mechanism of action (MOA) of the anti-fungal function of Zinc Pyrithione (ZPT) as an anti-dandruff active agent has demonstrated that iron chelation is a major element of the biological activity. Based on these and similar observations, the use of appropriate iron chelators in combination with ZPT is a key insight to developing significantly more effective anti-dandruff formulas.

Zinc Pyrithione (ZPT) is a common anti-dandruff active used in shampoos and other treatments. Mechanistic work has provided elements of its action such as the importance of chelating iron as an anti-fungal strategy. Other parameters identified that may affect the activity of an iron chelator include hydrophobicity which may be an element for compatibility with ZPT such that relatively low Zn affinity is needed.

By utilizing the new MOA understanding of ZPT to achieve increased anti-fungal activity, the present invention will deliver compositions and products with superior anti-dandruff performance.

SUMMARY OF THE INVENTION

The present invention is directed to a personal care composition comprising an effective amount of zinc pyrithione; an effective amount of an iron chelator or a material which chelates iron wherein the iron chelator or the material which chelates iron is selected from the group consisting of EDDHMA Zinc salt, Maltol Zinc salt, EDDHA Zinc salt, Benzohydroxamic acid Zinc salt, Thiomaltol Zinc salt and 1,2-Dimethyl-3-hydroxy-4-pyridone Zinc salt; wherein the Zinc salt of EDDHA and EDDHMA has stoichiometry zinc to ligand of 1:1 or 2:1; wherein EDDHA is selected from the group consisting of o,o-EDDHA (ethylenediamine-N,N'-di-(2-hydroxyphenyl acetic acid), and o,p-EDDHA (ethylenediamine-N-(2-hydroxyphenyl acetic acid)-N'-(4-hydroxyphenylacetic acid)), wherein EDDHMA is selected from the group consisting of o,o-EDDHMA (ethylenediamine-N,N'-di-(2-hydroxy-4-methylphenyl acetic acid)), and o,o'-EDDHMA (ethylenediamine-N-(2-hydroxy-4-methylphenyl acetic acid)-N' -(2-hydroxy-6-methylphenylacetic acid)), wherein the combination of the iron chelator and the pyrithione or a polyvalent metal salt of a pyrithione has a fractional inhibitor concentration of less than or equal to 0.5 according to the Fractional Inhibitor Concentration Test Method as disclosed hereinafter.

DETAILED DESCRIPTION OF THE INVENTION

While the specification concludes with claims which particularly point out and distinctly claim the invention, it is believed the present invention will be better understood from the following description.

The present invention can comprise, consist of, or consist essentially of the essential elements and limitations of the invention described herein, as well as any of the additional or optional ingredients, components, or limitations described herein.

All percentages, parts and ratios are based upon the total weight of the compositions of the present invention, unless otherwise specified. All such weights as they pertain to listed ingredients are based on the active level and, therefore do not include carriers or by-products that may be included in commercially available materials.

The components and/or steps, including those which may optionally be added, of the various embodiments of the present invention, are described in detail below. The citation of any document is not to be construed as an admission that it is prior art with respect to the present invention.

All ratios are weight ratios unless specifically stated otherwise.

All temperatures are in degrees Celsius, unless specifically stated otherwise.

Except as otherwise noted, the articles "a", "an", and "the" mean "one or more".

Herein, "comprising" means that other steps and other ingredients which do not affect the end result can be added. This term encompasses the terms "consisting of' and "consisting essentially of'. The compositions and methods/processes of the present invention can comprise, consist of, and consist essentially of the essential elements and limitations of the invention described herein, as well as any of the additional or optional ingredients, components, steps, or limitations described herein.

Herein, "effective" means an amount of a subject active high enough to provide a significant positive modification of the condition to be treated. An effective amount of the subject active will vary with the particular condition being treated, the severity of the condition, the duration of the treatment, the nature of concurrent treatment, and like factors.

Herein, "personal care compositions" means products for and/or methods relating to treating hair (human, dog, and/or cat), including, bleaching, coloring, dyeing, conditioning, growing, removing, retarding growth, shampooing, styling; deodorants and antiperspirants; personal cleansing; color cosmetics; products, and/or methods relating to treating skin (human, dog, and/or cat), including application of creams, lotions, and other topically applied products for consumer use; and products and/or methods relating to orally administered materials for enhancing the appearance of hair, skin, and/or nails (human, dog, and/or cat); and shaving.

A. Iron Chelators

In the present invention, iron chelators may have, but not be limited to, the following characteristics:

1. An affinity for iron ions in either the ferrous (iron II) or ferric (III) forms;
2. Materials of Description 1 (above) that have a denticity of two or higher (denticity is the number of groups of a molecule that bind to the iron ion);
3. Chemical descriptions that are a subset of Description 2:

a. Either natural or synthetic (e.g., DFO, DFT) materials;
b. Materials of the following chemical classes:

i. Catechols and phenols
ii. Hydroxamates (desferrioxamine (DFO))
iii. Thiohydroxamates
iv. Hydroxypyridones (CP20, piroctone, ciclopirox, HP-101)
v. Hydroxythiopyridones
vi. Hydroxypyridinethiones
vii Aminocarboxylates (EDTA, DTPA)
viii Pyridines (2,2'-bipyridine, 1,10-phenatholine, TPEN)
ix. Hydroxycarboxylates
x. Aroylhydrazones (PIH)
xi. Hydroxyquinolines (8-hydroxyquinoline)
xii. Hydroxypyrones (maltol, ethyl maltol)
xiii. Hydroxythiopyrones

and molecules representing combinations of these chemical classes.

N-Hydroxy-6-octyloxypyridine-2(1H)one, ethanolamine salt, (HP-101) as supplied from Arch Chemicals, Inc., is part of the N-Hydroxypyridones. The N-Hydroxypyridones have alkyl ether substitutions at the 6-position as free acids, ethanolamine salts and metal salts such as zinc, N-Hydroxy-6-octyloxypyridine-2(1H)one, zinc salt. The alkyl ether substituent is from 2-22 carbons in length, either linear or branched.

For the zinc salts of materials such as EDDHA and EDDHMA, from Akzo-Nobel, the stoichiometry may be 1:1 zinc to ligand or 2:1. The chelating agents EDDHA, EDDHMA are intended to cover all their isomeric forms. Non-limiting examples of chelating agents covered by the term EDDHA include o,o-EDDHA (ethylenediamine-N,N'-di(2-hydroxyphenyl acetic acid), and o,p-EDDHA - ethylenediamine-N-(2-hydroxyphenyl acetic acid)-N'-(4-hydroxyphenyl acetic acid) and examples of the chelating agent EDDHMA include o,o-EDDHMA - ethylenediamine-N,N'-di(2-hydroxy-4-methylphenyl acetic acid), and o,o'-EDDHMA - ethylenediamine-N-(2-hydroxy-4-methylphenyl acetic acid)-N'-(2-hydroxy-6-methylphenyl acetic acid).

B. Pyrithione or a Polyvalent metal salt of Pyrithione

The personal care composition comprises pyrithione or a polyvalent metal salt of pyrithione being zinc pyrithione. Any form of zinc pyrithione salts may be used, including platelet and needle structures.. For use herein is the zinc salt of 1-hydroxy-2-pyridinethione (known as "zinc pyrithione" or "ZPT"); in yet a further embodiment, ZPT in platelet particle form, wherein the particles have an average size of up to 20μm, and in an embodiment have an average size of up to 5μm, and yet in a further embodiment have an average size of up to 2.5μm.

Pyridinethione anti-microbial and anti-dandruff agents are described, for example, in U.S. Pat. No. 2,809,971; U.S. Pat. No. 3,236,733; U.S. Pat. No. 3,753,196; U.S. Pat. No. 3,761,418; U.S. Pat. No. 4,345,080; U.S. Pat. No. 4,323,683; U.S. Pat. No. 4,379,753; and U.S. Pat. No. 4,470,982.

It is further contemplated that when ZPT is used as the anti-microbial particulate in the anti-microbial compositions herein, that an additional benefit of hair growth or re-growth may be stimulated or regulated, or both, or that hair loss may be reduced or inhibited, or that hair may appear thicker or fuller.

Zinc pyrithione may be made by reacting 1-hydroxy-2-pyridinethione (i.e., pyrithione acid) or a soluble salt thereof with a zinc salt (e.g. zinc sulfate) to form a zinc pyrithione precipitate, as illustrated in U.S. Patent No. 2,809,971.

Embodiments include from 0.01% to 5% of a pyrithione or polyvalent metal salt of a pyrithione; and from 0.01% to 5% of an iron chelator; more in an embodiment, each from 0.1% to 2%.

In embodiments having a pyrithione or polyvalent metal salt of pyrithione, the ratio of iron chelator to pyrithione or a polyvalent metal salt of pyrithione may be in the range of 1:10 to 10:1.

C. Hydrophobicity

In one embodiment of the present invention, the iron chelator is hydrophobic. Sufficient hydrophobicity is required to increase the probability of the material traversing the cell membrane of the fungus to exert its effect. AlogP can be a measure of material hydrophobicity, which is calculated based on a chemical fragment approach. The more positive the number, the more hydrophobic the molecule, a minimum of which is believed to be needed to enhance membrane permeability. Intrinsic hydrophobicity can be calculated as AlogP for the isolated molecule. Materials with charged groups tend to be much less hydrophobic, often even becoming highly water soluble.

In the case where molecules are charged (especially anionic) and tend not to be hydrophobic enough to be cell permeable, an approach is to neutralize the charge by making certain metal ion salts, resulting in increased hydrophobicity and membrane permeability. The algorithms for calculating log P are not effective for metal salts and thus such differences must be measured. For sufficient hydrophobicity of a parent anionic material, the metal-ligand ratio should be sufficient to result in a charge neutral complex salt. Further, the metal used should be such that it can easily be replaced by metals such as iron once entering the cell. Desirable metal salts, then, should have a lower affinity for the ligand than iron; zinc salts are particularly preferred in this regard, though other salts are possible as long as they bind less strongly than iron.

In an embodiment of the present invention, a personal care composition comprises an iron chelator wherein the iron chelator is a metal salt of zinc.

In a further embodiment of the present invention, the iron chelator has an AlogP value of greater than or equal to 0.4, and in an embodiment has an AlogP value of greater than or equal to 0.5, and in a further embodiment has an AlogP value of greater than or equal to 0.6, and in yet a further embodiment has an AlogP value of greater than or equal to 0.7,

D. IRON AFFINITY - log $K_1$

The strength of the association between a ligand and metal, in this case iron, can be termed iron affinity. Without being bound by theory, materials that meet a minimum iron affinity have the potential to disrupt iron cellular metabolism thereby increasing anti-microbial activity when combined with other materials that are stressing cellular physiology.

Affinity between a metal (M) and ligand (L) can be measured by the stepwise association constant, $K_1$ which describes the following equilibrium:

$$\mathrm{M} + \mathrm{L} \rightleftharpoons \mathrm{ML};\ K_1 = \frac{[\mathrm{ML}]}{[\mathrm{M}][\mathrm{L}]}$$

The affinity constant is conveniently expressed as the logarithm (log $K_1$) and the larger the magnitude of this number, the stronger the association between the metal (iron ions in this case) and ligand.

In an embodiment of the present invention, a log $K_1 > 3.5$ for demonstrating anti-fungal activity, in a further embodiment a log $K_1 > 3.7$, and in yet a further embodiment a log $K_1 > 3.8$.

E. Intrinsic Anti-Malassezia Potency- IC50

The IC50 value is the inhibitor concentration that causes growth inhibition so that the culture optical density has increased ½ as much as that of the untreated control.

To measure the growth inhibition properties of test materials against *Malassezia,* we carried out the following protocol. We cultured *Malassezia furfur* in 100 ml mDixon medium (per one liter: 36 g malt extract (Difco 0186-17), 20 g ox bile (Fluka 70168), 10 ml Tween 40 (Aldrich 27435-6), 6 g peptone (Difco 0118-17), 2 ml oleic acid (Baker 2114-01), and 2 ml glycerol (Sigma G-7893). The pH is adjusted to 6.0 using IN HCl (Baker 562-2). The media is autoclaved, and then 5 ml 0.1g/ml chloramphenicol is added.

Starting cultures are prepared by incubating with shaking at 31°. Cells (5 ml of culture) are collected by centrifugation and suspended in 50 ml fresh mDixon medium. Cells (290 μl) are mixed with inhibitor (10 μl) in a deep well polypropylene plate (Beckman 267007) and sealed with a semipermeable membrane (Excel Scientific BS-25). The plates are shaken vigorously on a Heidolph Titramax 100 overnight at 31°. To increase the humidity, water-soaked cotton batting is placed over the plates. Some (200 μl) of the culture is transferred to a Costar 3596 plate, and the optical density (600 nm) is measured.

As a control, the optical density of untreated *M. furfur* is measured after overnight incubation. As another control, the optical density of the starting culture is measured. The difference between these optical densities represents the amount of growth of *M. furfur.*

Table 1.

| Material | Hydrophobicity, ALogP* | Iron Affinity, log $K_1$** $Fe^{2+}$, pH = 7.4 | Intrinsic Anti-Malassezia Potency (IC50, μM) |
|---|---|---|---|
| Zinc Pyrithione | 1§ | 4.3 (c) | 15.0 |
| 5,7-dichloro-8-hydroxyquinoline N-Hydroxy-6-octyloxypyridine- | 3.1 | 7.2 (b) | 23.4 |
| 2(1H)one, ethanolamine salt (HP-101, Arch Chemical) | 3.5 | 5.4 (d) | 54.6 |
| 1,5-Diphenyl-1,2,4,5-tetraazapent-1-en-3-thione (Diphenylthiocarbazone, Dithizone) | 5.0 | 6.5 (a) | 62.5 |
| 1-Hydroxy-4-(1-methylethyl)cyclohepta-3,5,7-trien-2-one (beta-Isopropyltropolone) | 1.9 | 4.9 (c) | 65 |
| 5-Chloro-8-hydroxy-7-iodoquinoline (Clioquinol) | 3.0 | 7.0 (b) | 65.5 |
| N,N,N',N-tetrakis(2-pyridylmethyl) ethylenediamine (TPEN) | 3.1 | 14.2 (a) | 71 |
| 2,2':6',2"-terpyridine | 3.1 | 5.0 (c) | 86 |
| Pyrithione | 0.7 | 4.3 (c) | 90.0 |
| 8-hydroxyquinoline | 1.8 | 4.9 (c) | 137.8 |
| 1,10-Phenanthroline | 2.2 | 5.9 (a) | 166.5 |
| 1-(2-Thiazoylazo)-2-naphthol | 3.9 | 6.6 (c) | 250 |
| trans-1,2-Cyclohexylenedinitrilotetraacetic acid (CDTA) | -6.3 | 14.0 (a) | 500 |
| kojic acid | -1.1 | 3.7 (c) | 704.0 |
| 3-Hydroxy-1,2-dimethyl-4(1H)-pyridone (Deferiprone) | -0.1 | 3.9 (c) | 719.0 |
| maltol | -0.2 | 6.4 (c) | 793.0 |
| 2,9-dimethyl-1,10-phenanthroline | 2.8 | 3.3 (c) | >480 |
| 1,4,7,10,13,16-Hexaazacyclooctadecane ([18]aneN6) | -2.4 | 13.3 (e) | >500 |
| 1,4,8,11-Tetraazacyclotetradecane-N,N',N",N"'-tetraacetic acid (TETA) | -13.5 | 9.9 (a) | >500 |
| 1-Hydroxy-1,2-dihydropyridin-2-one | -0.3 | 4.2 (c) | >500 |
| 2-(2-aminoethyl)-pyridine | 0.2 | 5.2 (d) | >500 |
| beta-Alanyl-L-histidine (L-Carnosine) | -4.5 | 1.3 (c) | >500 |
| Ethylenediiminodi-2-butanoic acid | -5.0 | 6.3 (c) | >500 |
| L-2-Amino-3-phosphopropanoic acid (Phosphoserine) | -4.1 | 2.3 (c) | >500 |
| N,N-Bis(2-hydroxyethyl)glycine (Bicine) | -4.3 | 3.6 (c) | >500 |
| Salicylaldoxime | 1.4 | 0.7 (d) | >500 |
| Salicylic acid | 1.2 | -3.0 (e) | >500 |
| Ethylenediaminetetraacetic acid (EDTA) | -7.7 | 12.2 (a) | >500 |

(continued)

| Material | Hydrophobicity, ALogP* | Iron Affinity, log $K_1$** $Fe^{2+}$, pH = 7.4 | Intrinsic Anti-Malassezia Potency (IC50, $\mu$M) |
|---|---|---|---|
| 4-(2-thiazolylazo)-resorcinol | 2.7 | 3.2 (a) | > 900 |

§Measured, not calculated
*Calculated by published methods,
see AK Ghose J Phys Chem A
**1998,** *102*, 3762.

" Data obtainerd from sources as marked:

(a) NIST Standard Reference Database 46, Version 8.0: NIST Critical Selected Stability Constants of Metal Complexes Database. Arthur E. Martell & Robert M. Smith, 2004.
(b) IUPAC Stability Constant Database.
(c) Data interpolated from the correlation between $Zn^{2+}$ and $Fe^{2+}$ data from sources (a) and (b) with a a regression equation of y=0.8646x-0.2482 with $R^2$=0.91.
(d) Data interpolated from the correlation between $Fe^{3+}$ and $Fe^{2+}$ data from sources (a) and (b) with a regression equation of y=0.6637x-2.056 with $R^2$=0.90.
(e) Data interpolated from the correlation between $Cu^{2+}$ and $Fe^{2+}$ data from sources (a) and (b) with a regression equation of y=0.7003x-1.0113 with $R^2$=0.78.

F. Fractional Inhibitor Concentration (FIC)

Fractional Inhibitor Concentration (FIC) is a conventional methodology for evaluating antimicrobial interactions. In an embodiment of the present invention, an FIC is used to determine combinatorial effects of two chemicals on anti-*Malassezia* activity in a tissue culture setting.

**I.** Method Overview:

1. Each FIC assay is run in duplicate.
2. Low levels of *M. furfur* 7982 cells are inoculated into each well of a 96 well plate.
3. Chemical #1 (in our assays, this is always ZPT) is titrated (2x concentration reductions) from right to left across each row of the plate, starting at 12.5ppm final concentration and ending at 0ppm.
4. Chemical #2 is titrated (2x concentration reduction) down each column of the plate, starting at either 400ppm or 800ppm final concentration (depending on potency) and ending at 0ppm.
5. Plates are incubated at 33°C, 60%RH for 2 days with agitation.
6. Optical densities (OD's) are then determined using a spectrophotometer.

**II.** Data Analysis:

1. Inhibition of growth is determined if the OD <=0.6 (this indicates that growth in the well did not achieve at least 50% of the maximal level possible), and the well is labeled with a '-'.
2. Lack of inhibition of growth is determined if the OD>0.6, and the well is labeled with a '+'.
3. The lowest concentration value of chemical #1, both by itself and with the lowest concentration of chemical #1 which boosts chemical #2's potency, that inhibit growth are determined (see Appendix 1).
4. Likewise, the lowest concentration of chemical #2, both by itself and with the lowest concentration of chemical #2 that boosts chemical #1's potency, that inhibit growth are determined (see Appendix 1).
5. The FIC value for the duplicate plates is determined using the calculations below:

$$FIC = \frac{\text{lowest inhibitory dose of Chemical \#1 by itself}}{\text{lowest inhibitory dose of Chemical \#1 in a well that also contains Chemical \#2}} + \frac{\text{lowest inhibitory dose of Chemical \#2 by itself}}{\text{lowest inhibitory dose of Chemical \#2 in a well that also contains Chemical \#1}}$$

$$\text{ave FIC VALUE} = \frac{(\text{FIC value for plate 1} + \text{FIC value for plate 2})}{2}$$

6. The average FIC value is then determined, and the combinatorial effect of the two chemicals is classified according to the chart below:

| ave FIC VALUE | Combinatorial Effect |
|---|---|
| <=0.5 | Synergistic |
| >0.5-1.0 | Additive |
| >1.0-<=4.0 | Indifferent |
| >4 | Antagonistic |

Appendix 1:

Below is an example process of how to interpret a hypothetical FIC, where all numerical values represent final concentrations of the tested chemicals in parts per million

| Chemical #2 | 0.00 | 0.20 | 0.39 | 0.78 | 1.56 | 3.13 | 6.25 | 12.50 |
|---|---|---|---|---|---|---|---|---|
| 400.00 | ▤ - | - | - | - | - | - | - | - |
| 200.00 | + | ▥ + | - | - | - | - | - | - |
| 100.00 | + | + | - | - | - | - | - | - |
| 50.00 | + | + | - | - | - | - | - | - |
| 25.00 | + | + | + | - | - | - | - | - |
| 12.50 | + | + | + | + | - | - | - | - |
| 6.25 | + | + | + | + | + | - | - | - |
| 3.13 | + | + | + | + | + | ▨ - | - | - |
| 1.56 | + | + | + | + | + | + | - | - |
| 0.78 | + | + | + | + | + | + | - | - |
| 0.39 | + | + | + | + | + | + | - | - |
| 0.20 | + | + | + | + | + | + | ▦ - | - |

Chemical #1

Per the instructions noted in section II.1, each well has been labeled either '+' or '-'. The horizontal lined well indicates the lowest value of chemical #2 by itself (read to the left as 400.00). The diagonally lined well indicates the lowest value of chemical #2 that provides a boost to chemical #1's potency (read to the left is 3.13). The dotted well indicates the lowest value of chemical #1 by itself (read downward as 6.25). The vertical lined well indicates the lowest value of chemical #1 that provides a boost to chemical #2's potency (read downward as 0.20).

Per the instructions noted in section II.5, the FIC value is calculated by the following equation:

$$(0.20/6.25) + (3.13/400.00) = 0.04$$

Per the instructions noted in section II.1, this plate can be been run in duplicate, and here it is assumed to have given the same result.

Per the instructions noted in section II.5, the average FIC value is calculated by the following equation:

$$(0.04+0.04)/2 = 0.04$$

Per the instructions noted in section II.6, given the average FIC value of 0.04, the combinatorial effect of these two chemicals is determined to be synergistic.

Table 2.
Examples of Additivity and Synergy for Chelators with ZPT

| Material | FIC* |
|---|---|
| EDDHMA, Zinc Salt (1) | 0.1 |
| Maltol, Zinc salt | 0.1 |
| EDDHA, Zinc Salt (1) | 0.2 |
| Benzohydroxamic acid, Zinc Salt | 0.3 |
| Thiomaltol, Zinc salt | 0.5 |
| N-Hydroxy-6-octyloxypyridine-2(1H)one, ethanolamine salt (HP-101) (2) | 0.62 |
| N-Hydroxy-6-octyloxypyridine-2(1H)one, zinc salt (HP-100 Zn) (2) | 0.7 |
| 1,2-Dimethyl-3-hydroxy-4-pyridone, Zinc salt | 0.5 |
| Thiomaltol | 0.8 |
| N,N-Naphthaloylhydroxylamine | 0.8 |

(1) From Akzo-Nobel
(2) From Arch Chemicals, Inc.
*Fractional Inhibitory Concentration wherein FIC ≤ 0.5 represents synergistic activities and 0.5 < FIC ≤ 1.0 represents additivity (FIC's above 1.0 are either indifferent or antagonistic). Reference: R. Bharadwaj et al Indian J Pharmacol 2003, 35, 220.

The hydrophobicity and iron affinity data of Table 1 are arranged in approximate order of decreasing intrinsic anti-fungal potency (measured as $IC_{50}$). The intrinsic anti-fungal potency is being used as a predictor of the potential to have either synergistic or additive anti-microbial benefits when combined with another anti-fungal agent.

The data of Table 1 clearly show both hydrophobity and iron affinity parameters must meet certain minimal criteria to result in appreciable independent anti-fungal activity. For example, the chemically related 1,10-phenanthroline and 2,9-dimethyl phenanthroline have substantially different intrinsic anti-fungal potencies that can be interpreted as being due to 2,9-diphenyl phenanthroline having a much lower iron affinity than the parent phenanthroline molecule.

Note that very common iron chelators such as EDTA are observed not to be effective, this is likely due to the very low hydrophobicity which, without being bound by theory, likely limits the permeability into the cell, where the site of action of iron binding is thought to occur.

When evaluating the additivity and synergism data of Table 2, it is clear that there can be very strong interactions with, in this example, zinc pyrithione to increase anti-fungal potency of the combination. In these examples, it becomes clear that some materials can be substantially improved by making certain metal salts of the materials. For example, maltol (from Table 1) is too hydrophilic to be an effective anti-fungal material independently. However, its zinc salt is considerably more hydrophobic (cannot be calculated by the AlogP method) enabling it to potentiate the activity of zinc pyrithione (see table 2, FIC of 0.1).

Anionic iron binding materials have the potential, in general, to be made more hydrophobic, and thereby increase cellular permeability, by making certain metal salts of these materials. While zinc is most preferred, other metals, such as calcium, magnesium and barium are possible.

The combination of an iron chelator and pyrithione or a polyvalent metal salt of a pyrithione has a fractional inhibitor concentration of less than or equal to 0.5 according to the Fractional Inhibitor Concentration Test Method as disclosed herein.

Additional information regarding fractional inhibitor concentration is found in AN INVITRO STUDY TO EVALUATE THE SYNERGISITC ACTIVITY OF NORFLOXACIN AND METRONIDAZOLE, R. Bharadwaj, et al., Indian J Pharmacol 2003, 35: 220-226.

In an embodiment of the present invention, the personal care composition may further comprise a surfactant. In a further embodiment, the composition of the present invention may include a detersive surfactant. The detersive surfactant component is included to provide cleaning performance to the composition. The detersive surfactant component in turn comprises anionic detersive surfactant, zwitterionic or amphoteric detersive surfactant, or a combination thereof. Such surfactants should be physically and chemically compatible with the essential components described

**EP 2 632 470 B1**

herein, or should not otherwise unduly impair product stability, aesthetics or performance. Suitable anionic detersive surfactant components for use in the composition herein include those which are known for use in hair care or other personal care cleansing compositions. Nonlimiting examples of anionic surfactants are described in U.S. Pat. Nos. 2,486,921; 2,486,922; 2,396,278 and 3,332,880. The concentration of, for example, an anionic surfactant component in the composition should be sufficient to provide the desired cleaning and lather performance, and generally range from 5% to 50%. Non limiting examples of other anionic, zwitterionic, amphoteric or optional additional surfactants suitable for use in the compositions are described in McCutcheon's, Emulsifiers and Detergents, 2002 Annual, published by M. C. Publishing Co., and U.S. Pat. Nos. 3,929,678, 2,658,072; 2,438,091; 2,528,378.

In a further embodiment, the composition of the present invention may be in the form of a topical compositions, which includes a topical carrier. In an embodiment, the topical carrier is selected from a broad range of traditional personal care carriers depending on the type of composition to be formed. By suitable selections of compatible carriers, it is contemplated that such a composition is prepared in the form of daily skin or hair products including conditioning treatments, cleansing products, such as hair and/or scalp shampoos, body washes, hand cleansers, water-less hand sanitizer/cleansers, facial cleansers and deodorants.

The compositions of the present invention may further comprise one or more optional components known for use in hair care or personal care products, provided that the optional components are physically and chemically compatible with the essential components described herein, or do not otherwise unduly impair product stability, aesthetics or performance. Individual concentrations of such optional components may range from 0.001% to 10%.

Non-limiting examples of optional components for use in the composition include cationic polymers, cationic surfactants, conditioning agents (hydrocarbon oils, fatty esters, silicones), anti dandruff agents, suspending agents, viscosity modifiers, dyes, nonvolatile solvents or diluents (water soluble and insoluble), pearlescent aids, foam boosters, additional surfactants or nonionic cosurfactants, pediculocides, pH adjusting agents, perfumes, preservatives, chelants, proteins, skin active agents, sunscreens, UV absorbers, vitamins, minerals, herbal/fruit/food extracts, sphingolipids derivatives or synthetical derivative, and clay. Nonlimiting examples of the optional components are found in International Cosmetic Ingredient Dictionary, Fifth Edition, 1993, and CTFA Cosmetic Ingredient Dictionary, 3rd edition, edited by Estrin, Crosley, and Haynes, (The Cosmetic, Toiletry, and Fragrance Association, Inc., Washington, D.C. (1982)).

**Claims**

1.  A personal care composition comprising:

    a) an effective amount of Zinc Pyrithione;
    b) an effective amount of an iron chelator or a material which chelates iron, wherein the iron chelator or a material which chelates iron is selected from the group consisting of EDDHMA Zinc salt, Maltol Zinc salt, EDDHA Zinc salt, Benzohydroxamic acid Zinc salt, Thiomaltol Zinc salt and 1,2-Dimethyl-3-hydroxy-4-pyridone Zinc salt; wherein the Zinc salt of EDDHA and EDDHMA has a stoichiometry zinc to ligand of 1:1 or 2:1; wherein EDDHA is selected from the group consisting of o,o-EDDHA (ethylenediamine-N,N'-di(2-hydroxyphenyl acetic acid), and o,p-EDDHA (ethylenediamine-N-(2-hydroxyphenyl acetic acid)-N' -(4-hydroxyphenylacetic acid)); wherein EDDHMA is selected from the group consisting of o,o-EDDHMA (ethylenediamine-N,N' -di(2-hydroxy-4-methylphenyl acetic acid)), and o,o'-EDDHMA (ethylenediamine-N -(2-hydroxy -4-methylphenyl acetic acid)-N' -(2-hydroxy-6-methylphenylacetic acid)); wherein the combination of the iron chelator and the pyrithione or a polyvalent metal salt of a pyrithione has a fractional inhibitor concentration of less than or equal to 0.5 according to the Fractional Inhibitor Concentration Test Method as disclosed herein in the present application.

2.  A personal care composition according to claim 1 wherein the pyrithione or a polyvalent metal salt of a pyrithione is present from 0.01% to 5%.

3.  A personal care composition according to any preceding claims wherein the iron chelator is present from 0.01% to 5%.

4.  A personal care composition according to any preceding claims wherein the pyrithione or a polyvalent metal salt of a pyrithione is present from 0.1% to 2% and the iron chelator is present from 0.1% to 2%.

5.  A personal care composition according to any preceding claims, wherein the ratio of iron chelator to pyrithione or a polyvalent metal salt of pyrithione is in the range of 1:10 to 10:1.

6.  A personal care composition according to claim 1, wherein the Zinc Pyrithione is in platelet particle form, wherein the particles have an average size of up to 20μm.

7. A personal care composition according to claim 6, wherein the particles have an average size of up to 5μm, preferably wherein the particles have an average size of up to 2.5μm.

8. A personal care composition according to any preceding claims, wherein the personal care composition comprises a surfactant.

9. A personal care composition according to claim 8, wherein the surfactant of the personal care composition comprises a detersive surfactant.

10. A personal care composition according to claim 9, wherein the detersive surfactant of the personal care composition comprises anionic detersive surfactant, zwitterionic or amphoteric detersive surfactant, or a combination thereof.

**Patentansprüche**

1. Körperpflegezusammensetzung, umfassend:

a) eine wirksame Menge an Zinkpyrithion;
b) eine wirksame Menge eines Eisenchelatbildners oder eines Materials, das mit Eisen einen Chelatkomplex bildet; wobei der Eisenchelatbildner oder ein Material, das mit Eisen einen Chelatkomplex bildet, ausgewählt ist aus der Gruppe bestehend aus EDDHMA-Zinksalz, Maltol-Zinksalz, EDDHA-Zinksalz, Benzohydroxamin-Zinksalz, Thiomaltol-Zinksalz und 1,2-Dimethyl-3-hydroxy-4-pyridon-Zinksalz;
wobei das Zinksalz von EDDHA und EDDHMA eine Stöchiometrie von Zink zu Ligand von 1:1 oder 2:1 aufweist;
wobei EDDHA ausgewählt ist aus der Gruppe bestehend aus o,o-EDDHA (Ethylendiamine-N,N'-di(2-hydroxyphenylessigsäure) und o,p-EDDHA (Ethylendiamin-N-(2-hydroxyphenylessigäsure)-N'-(4-hydroxyphenylessigsäure));
wobei EDDHMA ausgewählt ist aus der Gruppe bestehend aus o,o-EDDHMA (Ethylendiamin-N,N'-di(2-hydroxy-4-methylphenylessigsäure)) und o,o'-EDDHMA (Ethylendiamin-N-(2-hydroxy-4-methylphenylessigsäure)-N'-(2-hydroxy-6-methylphenylessigsäure));
wobei die Kombination aus dem Eisenchelatbildner und dem Pyrithion oder einem mehrwertigen Metallsalz von Pyrithion gemäß dem hierin in der vorliegenden Anmeldung offenbarten Verfahren zum Testen auf die Konzentration an fraktionellem Inhibitor eine Konzentration an fraktionellem Inhibitor von höchstens 0,5 aufweist.

2. Körperpflegezusammensetzung nach Anspruch 1, wobei das Pyrithion oder ein mehrwertiges Metallsalz eines Pyrithions 0,01 % bis 5 % ausmacht.

3. Körperpflegezusammensetzung nach einem der vorstehenden Ansprüche, wobei der Eisenchelatbildner 0,01 % bis 5 % ausmacht.

4. Körperpflegezusammensetzung nach einem der vorstehenden Ansprüche, wobei das Pyrithion oder ein mehrwertiges Metallsalz eines Pyrithions von 0,1 % bis 2 % ausmacht und der Eisenchelatbildner von 0,1 % bis 2 % ausmacht.

5. Körperpflegezusammensetzung nach einem der vorstehenden Ansprüche, wobei das Verhältnis von Eisenchelatbildner zu Pyrithion oder mehrwertigem Metallsalz von Pyrithion im Bereich von 1:10 bis 10:1 liegt.

6. Körperpflegezusammensetzung nach Anspruch 1, wobei das Zinkpyrithion in Form von plättchenförmigen Teilchen vorliegt, wobei die Teilchen eine durchschnittliche Größe von bis zu 20 μm aufweisen.

7. Körperpflegezusammensetzung nach Anspruch 6, wobei die Teilchen eine durchschnittliche Größe von bis zu 5 μm aufweisen, wobei die Teilchen vorzugsweise eine durchschnittliche Größe von bis zu 2,5 μm aufweisen.

8. Körperpflegezusammensetzung nach einem der vorstehenden Ansprüche, wobei die Körperpflegezusammensetzung ein Tensid umfasst.

9. Körperpflegezusammensetzung nach Anspruch 8, wobei das Tensid der Körperpflegezusammensetzung ein reinigungsaktives Tensid umfasst.

**10.** Körperpflegezusammensetzung nach Anspruch 9, wobei das reinigungsaktive Tensid der Körperpflegezusammensetzung ein anionisches reinigungsaktives Tensid, ein zwitterionisches oder amphoteres reinigungsaktives Tensid oder eine Kombination davon umfasst.

**Revendications**

**1.** Composition de soins personnels comprenant :

> a) une quantité efficace de pyrithione de zinc ;
> b) une quantité efficace d'un agent chélatant du fer ou d'un matériau qui chélate le fer ; dans laquelle l'agent chélatant du fer ou un matériau qui chélate le fer est choisi dans le groupe constitué d'un sel de zinc d'EDDHMA, d'un sel de zinc de Maltol, d'un sel de zinc d'EDDHA, d'un sel de zinc benzohydroxamique, d'un acide de thiomaltol et d'un sel de zinc de 1,2-diméthyl-3-hydroxy-4-pyridone ;
> dans laquelle le sel de zinc d'EDDHA et d'EDDHMA a une stoechiométrie du zinc au ligand de 1:1 ou de 2:1 ;
> dans laquelle l'EDDHA est choisi dans le groupe constitué de o,o-EDDHA (éthylène-diamine-N,N'-di(acide 2-hydroxyphénylacétique), et de o,p-EDDHA (éthylène-diamine-N-(acide 2-hydroxyphénylacétique)-N'-(acide 4-hydroxyphénylacétique)) ;
> dans laquelle l'EDDHMA est choisi dans le groupe constitué de o,o-EDDHMA (éthylène-diamine-N,N'-di(acide 2-hydroxy-4-méthylphénylacétique)), et de o,o'-EDDHMA (éthylène-diamine-N-(acide 2-hydroxy-4-méthylphénylacétique)-N'-(acide 2-hydroxy-6-méthylphénylacétique)) ;
> dans laquelle la combinaison de l'agent chélatant du fer et de la pyrithione ou d'un sel métallique polyvalent d'une pyrithione a une concentration inhibitrice fractionnaire inférieure ou égale à 0,5 selon le procédé de test de concentration inhibitrice fractionnaire tel que décrit ici dans la présente demande.

**2.** Composition de soins personnels selon la revendication 1, dans laquelle la pyrithione ou un sel métallique polyvalent d'une pyrithione est présent à raison de 0,01 % à 5 %.

**3.** Composition de soins personnels selon l'une quelconque des revendications précédentes, dans laquelle l'agent chélatant du fer est présent à raison de 0,01 % à 5 %.

**4.** Composition de soins personnels selon l'une quelconque des revendications précédentes, dans laquelle la pyrithione ou un sel métallique polyvalent d'une pyrithione est présent à raison de 0,1 % à 2 % et l'agent chélatant du fer est présent à raison de 0,1 % à 2 %.

**5.** Composition de soins personnels selon l'une quelconque des revendications précédentes, dans laquelle le rapport de l'agent chélatant du fer à la pyrithione ou au sel métallique polyvalent de pyrithione est dans la plage de 1:10 à 10:1.

**6.** Composition de soins personnels selon la revendication 1, dans laquelle la pyrithione de zinc est sous forme de particules en plaquette, dans laquelle les particules ont une taille moyenne allant jusqu'à 20 μm.

**7.** Composition de soins personnels selon la revendication 6, dans laquelle les particules ont une taille moyenne allant jusqu'à 5 μm, de préférence dans laquelle les particules ont une taille moyenne allant jusqu'à 2,5 μm.

**8.** Composition de soins personnels selon l'une quelconque des revendications précédentes, où la composition de soins personnels comprend un agent tensioactif.

**9.** Composition de soins personnels selon la revendication 8, dans laquelle l'agent tensioactif de la composition de soins personnels comprend un agent tensioactif détersif.

**10.** Composition de soins personnels selon la revendication 9, dans laquelle l'agent tensioactif détersif de la composition de soins personnels comprend un agent tensioactif détersif anionique, un agent tensioactif détersif zwittérionique ou amphotère, ou une combinaison de ceux-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2809971 A
- US 3236733 A
- US 3753196 A
- US 3761418 A
- US 4345080 A
- US 4323683 A
- US 4379753 A
- US 4470982 A
- US 2486921 A
- US 2486922 A
- US 2396278 A
- US 3332880 A
- US 3929678 A
- US 2658072 A
- US 2438091 A
- US 2528378 A

### Non-patent literature cited in the description

- **AK GHOSE.** *J Phys Chem A,* 1998, vol. 102, 3762
- **R. BHARADWAJ et al.** *Indian J Pharmacol,* 2003, vol. 35, 220
- **R. BHARADWAJ et al.** AN INVITRO STUDY TO EVALUATE THE SYNERGISITC ACTIVITY OF NORFLOXACIN AND METRONIDAZOLE. *Indian J Pharmacol,* 2003, vol. 35, 220-226
- McCutcheon's, Emulsifiers and Detergents. M. C. Publishing Co, 2002
- International Cosmetic Ingredient Dictionary. 1993
- CTFA Cosmetic Ingredient Dictionary. The Cosmetic, Toiletry, and Fragrance Association, Inc, 1982